# EUROPÄISCHE PATENTANMELDUNG

(11) **EP 3 278 793 A1**
(43) Veröffentlichungstag der Anmeldung: **07.02.2018**
(21) Anmeldenummer: 16182785.2
(22) Anmeldetag: 04.08.2016
(51) Int. Cl.: A61K 31/00

(54) **ZUSAMMENSETZUNG ZUR BEHANDLUNG VON VEISALGIA**

(71) Anmelder: ppsph. GmbH, 8003 Zürich (CH)
(72) Erfinder: Schmidt, Pedro, 8003 Zürich (CH)
(74) Vertreter: Hepp Wenger Ryffel AG

(57) **Zusammenfassung**

Die Erfindung betrifft eine Zusammensetzung zur Anwendung bei der Therapie von Veisalgia. Die Zusammensetzung umfasst mindestens eine Zuckerverbindung, Mineralsalze, mindestens ein Verbindung zum Zellschutz, mindestens eine Verbindung zur Unterstützung der Zellfunktion, mindestens eine Verbindung zur Förderung der Entgiftung, mindestens einen Neurotransmitter, mindestens ein Spurenelement, Folsäure, und optional weitere Spurenelemente und optional ein stimulierendes Alkaloid. Die Zusammensetzung enthält kein Analgetikum. Die Erfindung betrifft auch eine Dosierung der Zusammensetzung, ein Therapiekit sowie ein Analgetikum.

## Beschreibung

Die Erfindung betrifft eine Zusammensetzung zur Anwendung bei der Therapie von Veisalgia, ein Therapiekit zur Anwendung bei der Behandlung von Veisalgia sowie ein Analgetikum zur Behandlung von Veisalgia gemäss den Oberbegriffen der unabhängigen Ansprüche.

Veisalgia ist die medizinische Bezeichnung für Alkoholintoxikation. Sie geht häufig mit einer Vielzahl von Symptomen einher, wie beispielsweise Kopfschmerzen, Übelkeit, Schwindel, Schlaflosigkeit, Dehydrierung und Müdigkeit. Die Ursachen dieser Symptome sind sehr komplex und nicht alle Details der im Körper ablaufenden Prozesse sind geklärt. Dennoch konnten einige wesentliche Prozesse, die zu den Symptomen von Veisalgia führen, identifiziert werden. Einen Beitrag zu den Symptomen liefern beispielsweise die Dehydrierung und die Demineralisierung bei starkem Alkoholkonsum. Verstärkt wird dieser Faktor noch dadurch, dass die Betroffen es häufig versäumen, dem Flüssigkeitsmangel durch die Zufuhr nichtalkoholischer Getränke entgegen zu wirken. Weitere Ursachen für die Symptome sind beispielsweise in einer Übersäuerung des Magens, Unterzuckerung, Acetaldehyd- und Acetat-Akkumulierung aufgrund des Alkoholmetabolismus, oxidativer Stress oder Abbau von NAD⁺ zu NADH, was den Abbau von Alkohol im Körper verlangsamt, zu finden. Welche Symptome jedoch am Ende tatsächlich auftreten und auch die Ausgeprägtheit der Symptome ist von vielen verschiedenen Faktoren abhängig, u.a. von der Menge an Alkohol oder Körpergrösse, Gewicht und Geschlecht der konsumierenden Person.

Es sind therapeutische Zusammensetzungen bekannt, welche die Symptome zumindest teilweise lindern sollen. US 3 829 569 beschreibt beispielsweise eine Zusammensetzung mit sechs essentiellen Inhaltsstoffen: ein Analgetikum, ein Antidepressivum, ein mildes Magenmittel, ein mildes Anästhetikum, ein Mittel zur Unterstützung des Metabolismus sowie ein Mittel gegen Übersäuerung. Das Mittel wird in Form von zwei gleichzeitig einzunehmenden Kapseln verabreicht, wobei beide Kapseln ein Analgetikum umfassen. Die Aufteilung in zwei Kapseln erfolgt aufgrund der hohen Dosierung der einzelnen Bestandteile. Die in US 3 829 569 angegebene Dosierung soll die Symptome von Veisalgia deutlich lindern. Es hat sich gezeigt, dass diese Darreichungsform erhebliche Gesundheitsrisiken birgt. Den Mangelerscheinungen, welche zur Ausbildung von Veisalgia beitragen, kann damit nicht entgegen gewirkt werden.

Eine weitere Zusammensetzung zur Behandlung der oben genannten Symptome wird auch in WO 87/01285 beschrieben, wobei auch hier in jedem Fall ein Analgetikum mit verabreicht wird.

Es ist daher eine Aufgabe der Erfindung, die Nachteile des Standes der Technik zu überwinden. Insbesondere ist es eine Aufgabe der Erfindung, eine Zusammensetzung zur Anwendung bei der Therapie von Veisalgia bereitzustellen, welche das Auftreten der Veisalgia-Symptome weitestgehend verhindert bzw. deutlich verringert. Die Zusammensetzung soll dabei sicher in der Anwendung sein. Es ist eine weitere Aufgabe der Erfindung, ein Therapiekit zur Behandlung von Veisalgia bereitzustellen, welches das Auftreten von Symptomen weitestgehend verhindert aber auch symptomatische Linderung ermöglicht. Es ist auch Aufgabe der Erfindung, ein Analgetikum zur Behandlung von Veisalgia zur gleichzeitigen oder zeitversetzten Einnahme mit einer weiteren Zusammensetzung bereitzustellen.

Diese Aufgabe wird durch die in den unabhängigen Patentansprüchen definierten Zusammensetzungen, Therapiekits sowie Analgetika gelöst.

Die Erfindung betrifft eine Zusammensetzung zur Anwendung bei der Therapie von Veisalgia.

Die Zusammensetzung umfasst mindestens eine Zucker oder Zuckerverbindung. Der Zucker oder die Zuckerverbindung sind vorzugsweise aus der Gruppe: Fruktose, Saccharose und Glucose, ausgewählt. Eine bevorzugte Glucose ist D-Glucose, auch bekannt als Dextrose. Auch Verbindungen aus verschiedenen Zuckern, beispielsweise Fruktose-Dextrose, sind denkbar. Durch die Zuckerverbindungen kann eine Unterzuckerung vermieden werden. Unterzuckerung kann zu einer Folge von negativen Prozessen führen, welche die Ausbildung von Veisalgia-Symptomen fördern, beispielsweise Müdigkeit, Schwäche und Kopfschmerzen. Diesen Symptomen wird durch die Zuckerzufuhr weitestgehend entgegen gewirkt.

Die Zusammensetzung umfasst Mineralsalze. Bevorzugte Mineralsalze sind insbesondere ein Kaliumsalz, ein Natriumsalz, ein Magnesiumsalz und ein Kalziumsalz. Besonders bevorzugt ist die Verwendung von Kaliumchlorid, Natriumcitrat, Magnesiumcarbonat und/oder Kalziumcarbonat. Dies hat den Vorteil, dass wichtige Mineralien, welche aufgrund des Alkoholkonsums aus dem Körper geschwemmt werden, diesem wieder zugeführt werden. Diese Mineralien sind besonders wichtig in Bezug auf das Nervensystem und das Muskelsystem und wirken daher dem Auftreten zahlreicher Veisalgia-Symptome ebenfalls entgegen. Es können aber auch verschiedene Salze eines Minerals in der Zusammensetzung kombiniert werden, beispielsweise Magnesiumcarbonat, Magnesiumhydroxid und Magnesiumoxid. Dadurch werden beispielsweise die säurebindenden Eigenschaften sowie die Bioverfügbarkeit eines Minerals verstärkt.

Die Zusammensetzung umfasst mindestens eine Verbindung zum Zellschutz. Eine solche Verbindung kann insbesondere ein Antioxidans sein. Es hat sich als vorteilhaft erwiesen, mindestens ein Antioxidans aus der folgenden Gruppe auszuwählen: Ascorbinsäure, Tocopherol, einem Glutathion-Präkursor, Glutathion und/oder Phospahtidylserin. Alternativ ist es auch möglich, eine Kombination von verschiedenen Antioxidantien zu verwenden. Als Glutathion-Präkursor kommen beispielsweise N-Acyl-L-Cystein, L-Cystein und/oder Glutaminsäure in Betracht. Antioxidantien zeichnen sich durch ihre Eigenschaften als Radikalfänger, Reduktionsmittel und pH-regulierenden Eigenschaften aus. Als besonders vorteilhaft hat sich eine Kombination aus Ascorbinsäure (Vitamin C), Tocopherol (Vitamin E) und N-acyl-L-cystein (NAC) erwiesen. Dadurch wird insbesondere oxidativer Stress minimiert und ein verbessertes Wohlfühlgefühl erreicht. Der neurologische Zellschutz wird beispielsweise durch die Zugabe von Phosphatidylserin begünstigt.

Die Zusammensetzung umfasst weiter mindestens eine Verbindung zur Unterstützung der Zellfunktion. Die Verbindung ist vorzugsweise ausgewählt aus der Gruppe: Nicotinamid (Vitamin B₃), Nicotinsäure, Riboflavin (Vitamin B₂), Pantothensäure (Vitamin B₅), Pyridoxin (Vitamin B₆), Thiamin (Vitamin B₁), Vitamin B₁₂. Besonders bevorzugt ist eine Kombination aus zwei oder mehreren dieser Verbindungen. Diese Verbindungen unterstützen den Alkoholabbau in den Zellen. Thiamin und Vitamin B₁₂ tragen zum neurozellulären Schutz bei.

Des Weiteren umfasst die Zusammensetzung mindestens eine Verbindung zur Förderung der Entgiftung. Diese Verbindung ist insbesondere ausgewählt aus der Gruppe: Betaine, Silybum marianum (Mariendistel), Zingiberis rhizoma (Ingwerwurzel). Es ist aber auch möglich, eine Kombination entgiftungsfördernder Verbindungen zu verwenden. Diese Verbindungen zeichnen sich dadurch aus, dass sie insbesondere Lebervergiftungen entgegenwirken können bzw. den Abbau von Giftstoffen in der Leber fördern.

Die Zusammensetzung weist mindestens einen Neurotransmitter auf. Der Neurotransmitter ist vorzugsweise ausgewählt aus der Gruppe: L-Tyrosin, L-Tryptophan, L-Phenylalanin, L-D-Phenylalanin (Racemat), L-Glutamin, Dihydromyricetine. Der Begriff "Neurotransmitter" schliesst auch die Vorläufer für Neurotransmitter ein, d.h. Stoffe, die im menschlichen Körper in den entsprechenden Neurotransmitter umgewandelt werden können. So kann L-Tryptophan in Serotonin und L-Tyrosine in Dopamin umgewandelt werden. Diese Verbindungen haben eine stimmungsaufhellende Wirkung.

Die Zusammensetzung umfasst mindestens ein Spurenelement, insbesondere Selen und/oder Zink; und Folsäure. Als besonders vorteilhaft hat sich die Verwendung aller drei Spurenelemente erwiesen. Optional können der Zusammensetzung noch weitere Spurenelemente hinzugefügt werden, beispielsweise Biotin, Mangan und/oder Molybdän. Spurenelemente sind für die Aufrechterhaltung einer Vielzahl von Prozessen im menschlichen Körper essentiell.

Es kann wünschenswert sein, der Zusammensetzung ein stimulierendes Alkaloid, insbesondere Koffein, zuzusetzen. Dies beugt insbesondere Müdigkeitserscheinungen vor.

Die Zusammensetzung enthält kein Analgetikum.

Eine solche Zusammensetzung zur Anwendung bei der Therapie von Veisalgia hat den Vorteil, dass sie dem menschlichen Körper wieder die Nährstoffe zuführt, welche durch den Alkoholkonsum verloren gehen oder aufgrund dessen in erhöhter Menge benötigt werden und damit die Symptome der Vergiftungserscheinung reduziert. Unter Nährstoffen werden dabei allgemein Verbindungen zur Aufrechterhaltung von gesundheitlich relevanten biologischen Prozessen im menschlichen Körper, insbesondere die hier genannten Verbindungen, verstanden. Diese Zusammensetzung hat darüber hinaus den Vorteil, dass Sie vorbeugend schon vor oder während des Alkoholkonsums eingenommen werden kann, da sie kein Analgetikum enthält und Nebenwirkungen mit dem Alkohol daher nicht auftreten.

Die Zusammensetzung kann mindestens ein weiteres Additiv ausgewählt aus der Gruppe: Aroma, Farbstoffe, Zerfallsbeschleuniger, Säuerungsmittel, enthalten. Als Zerfallsbeschleuniger sind Stoffe zu verstehen, die das spätere Zerfallen der Tablette verbessern. Bevorzugt wird Natriumhydrogencarbonat verwendet. Es sind aber auch Mais- und Kartoffelstärke oder Polyvinylpyrrolidon denkbar. Als Säuerungsmittel wird vorzugsweise Zitronensäure verwendet. Es können auch mehrere Additive verwendet werden.

Solche Additive können einerseits die Handhabung der Tablette vereinfachen und andererseits das Aussehen sowie Geschmacksempfinden verbessern.

Vorzugsweise liegt die Zusammensetzung als Tablette, Kapsel, Suspension, lösbares Pulver, Fertiggetränk oder Depotformulierung vor. Als besonders vorteilhaft hat sich die Verwendung der Zusammensetzung in Form von Brausetabletten oder lösliches Pulver herausgestellt. Die Brausetablette oder das Pulver werden dabei zur Einnahme vorzugsweise in 300 bis 400 mL Wasser gelöst. Die so vorliegende Zusammensetzung kann besonders leicht und schnell eingenommen werden. Das Lösen in Wasser hat zusätzlich den Vorteil, dass ein vorliegender Flüssigkeitsmangel ausgeglichen wird.

Die Erfindung betrifft weiter eine Zusammensetzung zur Anwendung bei der Therapie von Veisalgia. Die Zusammensetzung umfasst
- mindestens einen Zucker oder eine Zuckerverbindung, insbesondere ausgewählt aus der Gruppe: Fruktose, Saccharose und Glucose;
- Mineralsalze; insbesondere ein Kaliumsalz, ein Natriumsalz, ein Magnesiumsalz und ein Kalziumsalz; und ganz besonders bevorzugt Kaliumchlorid, Natriumcitrat, Magnesiumcarbonat und/oder Kalziumcarbonat;
- mindestens ein Verbindung zum Zellschutz, insbesondere einem Antioxidans ausgewählt aus der Gruppe: Ascorbinsäure, Tocopherol, einem Glutathion-Präkursor, insbesondere N-Acyl-L-Cystein und/oder L-Cystein; Glutathion und/oder Phospahtidylserin;
- mindestens eine Verbindung zur Unterstützung der Zellfunktion, insbesondere ausgewählt aus der Gruppe: Nicotinamid, Nicotinsäure, Riboflavin, Pantothensäure, Pyridoxin, Thiamin, Vitamin B₁₂;
- mindestens eine Verbindung zur Förderung der Entgiftung, insbesondere Betaine, Silybum marianum, Zingiberis rhizoma;
- mindestens einen Neurotransmitter, insbesondere ausgewählt aus der Gruppe: L-Tyrosin, L-Tryptophan, L-Phenylalanin, L-D-Phenylalanin (Racemat), L-Glutamin, Dihydromyricetine;
- mindestens ein Spurenelement, insbesondere Selen und/oder Zink;
- Folsäure,
- und optional: weitere Spurenelemente, insbesondere Biotin, Mangan und/oder Molybdän;
- optional: ein stimulierendes Alkaloid, insbesondere Koffein;
und wird mindestens zweimal nach dem Alkoholkonsum verabreicht.

Durch die mindestens zweimalige Einnahme ist gewährleistet, dass fehlende Nährstoffe wieder in ausreichenden Mengen dem Körper zugeführt werden. Durch die mindestens zweimalige Einnahme wird eine gesundheitsfördernde Nährstoffkonzentration aufrechterhalten. Die Beschwerden, welche mit Veisalgia einhergehen, werden gelindert.

Als besonders vorteilhaft hat sich erwiesen, wenn die Verabreichung der Zusammensetzung mindestens einmal direkt nach dem Alkoholkonsum und zwei weitere Male innerhalb von 12 Stunden erfolgt. Zwischen den Einnahmen sollte jeweils eine Pause von mindestens 2 Stunden liegen. Die Zeitspanne von der ersten Einnahme bis zur dritten Einnahme sollte also mindestens sechs Stunden und höchstens 12 Stunden betragen.

Diese Dosierung hat den Vorteil, dass der therapeutische Effekt auch bestehen bleibt, wenn es aufgrund von pharmakokinetischen Eigenschaften und biologischer Halbwertzeit zu einer vorzeitigen Erniedrigung der Nährstoffkonzentration kommt.

Die Zusammensetzung kann mindestens einmal zusätzlichen vor dem Alkoholkonsum oder während des Alkoholkonsums verabreicht werden. Alternativ ist es auch möglich, die Zusammensetzung mindestens einmal vor dem Alkoholkonsum und mindestens einmal während des Alkoholkonsums zu verabreichen.

Die Zusammensetzung kann somit schon vorbeugend bzw. begleitend eingenommen werden. Durch die Einnahme vor dem Alkoholkonsum ist gewährleistet, dass die Nährstoffreserven im Körper aufgefüllt sind. Die Einnahme während des Alkoholkonsums gewährleistet, dass die Nährstoffkonzentration auf kein gesundheitsgefährdendes Mass absinkt. Die Beschwerden, welche durch Veisalgia hervorgerufen werden, treten weniger stark in Erscheinung. Durch die Abwesenheit eines Analgetikums treten keine Nebenwirkung auf, welche auf die Wechselwirkung eines Analgetikums mit Alkohol zurückzuführen sind.

Die Zusammensetzung kann mindestens 4 Stunden nach dem Alkoholkonsum zusammen mit einem Analgetikum verabreicht werden. Nach 4 Stunden ist die Alkoholkonzentration im Blut deutlich reduziert, so dass die Einnahme eines Analgetikums weniger problematisch ist.

Die gleichzeitige Einnahme der Zusammensetzung und des Analgetikums hat den Vorteil, dass die therapeutische Wirkung erhöht wird.

Die Zusammensetzung kann
- 5 bis 30 g Zucker oder Zuckerverbindungen;
- 100 bis 1500 mg der Mineralsalze;
- 10 bis 900 mg Verbindungen zum Zellschutz;
- 3 bis 7 g der Neurotransmitter;
- 1 bis 30 mg Verbindungen zur Unterstützung der Zellfunktion;
- 200 bis 1200 mg Verbindungen zur Förderung von Entgiftung
- 1 bis 7 mg der Spurenelement und Folsäure; und
- optional: 2 bis 10 mg Biotin; 1 bis 6 mg Mangan; 50 bis 300 mg Molybdän; und/oder 20 bis 100 mg Koffein enthalten.

Diese Angaben beziehen sich dabei auf die einmalige Einnahme der Zusammensetzung. Insgesamt können pro Therapie bis zu 50 g Zucker oder Zuckerverbindungen, 6 g Mineralsalze, 6 g Verbindungen zum Zellschutz, 14 g Neurotransmitter, 500 mg Verbindungen zur Unterstützung der Zellfunktion, 6 g Verbindungen zur Förderung der Entgiftung, 1 mg Folsäure und 50 mg Spurenelemente eingesetzt werden.

Des Weiteren betrifft die Erfindung ein Therapiekit zur Anwendung bei der Behandlung von Veisalgia. Das Therapiekit umfasst mindestens zwei separat verabreichbare Komponenten. Eine erste Komponente ist eine Zusammensetzung aus
- mindestens einen Zucker oder eine Zuckerverbindung, insbesondere ausgewählt aus der Gruppe: Fruktose, Saccharose und Glucose;
- Mineralsalze; insbesondere ein Kaliumsalz, ein Natriumsalz, ein Magnesiumsalz und ein Kalziumsalz; und ganz besonders bevorzugt Kaliumchlorid, Natriumcitrat, Magnesiumcarbonat und/oder Kalziumcarbonat;
- mindestens ein Verbindung zum Zellschutz, insbesondere einem Antioxidans ausgewählt aus der Gruppe: Ascorbinsäure, Tocopherol; einem Glutathion-Präkursor, insbesondere N-Acyl-L-Cystein und/oder L-Cystein; Glutathion und/oder Phospahtidylserin;
- mindestens eine Verbindung zur Unterstützung der Zellfunktion, insbesondere ausgewählt aus der Gruppe: Nicotinamid, Nicotinsäure, Riboflavin, Pantothensäure, Pyridoxin, Thiamin, Vitamin B₁₂;
- mindestens eine Verbindung zur Förderung der Entgiftung, insbesondere Betaine, Silybum marianum, Zingiberis rhizoma;
- mindestens einen Neurotransmitter, insbesondere ausgewählt aus der Gruppe: L-Tyrosin, L-Tryptophan, L-Phenylalanin, L-D-Phenylalanin (Racemat), L-Glutamin, Dihydromyricetine;
- mindestens ein Spurenelement, insbesondere Selen und/oder Zink;
- Folsäure, und
- optional weitere Spurenelemente, insbesondere Biotin, Mangan und/oder Molybdän; und
- optional: ein stimulierendes Alkaloid, insbesondere Koffein
und enthält kein Analgetikum.

Die zweite Komponente ist ein Analgetikum und kann alternativ auch ein Antazidum und/oder ein stimulierendes Alkaloid, vorzugsweise Koffein, enthalten.

Die erste Komponente kann in einer einmalig einzunehmenden Dosierung zwischen 5 bis 30 g Zucker oder Zuckerverbindungen; 100 bis 1500 mg der Mineralsalze; 10 bis 900 mg Verbindungen zum Zellschutz; 3 bis 7 g der Neurotransmitter; 1 bis 30 mg Verbindungen zur Unterstützung der Zellfunktion; 200 bis 1200 mg Verbindungen zur Förderung von Entgiftung; 1 bis 7 mg der Spurenelemente und Folsäure. Optional kann die Zusammensetzung 2 bis 10 mg Biotin und/oder 1 bis 6 mg Mangan und/oder 50 bis 300 mg Molybdän und/oder 20 bis 100 mg Koffein enthalten.

Die zweite Komponente kann in einer einmalig einzunehmenden Dosierung 100 bis 1000 mg des Analgetikums umfassen. Optional kann die zweite Komponente auch 100 bis 1000 mg eines Antazidums und/oder 20 bis 100 mg eines stimulierenden Alkaloids enthalten.

Das Kit zeichnet sich dadurch aus, dass die Zusammensetzung sowie das Analgetikum für die Behandlung von Veisalgia gleichzeitig und in ausreichenden Mengen zur Verfügung stehen. Aber auch eine separate Einnahme der Komponenten ist möglich. Auch kann eine Komponente bei Nicht-Bedarf weggelassen werden. Mit dem Therapiekit kann die Behandlung dem Schweregrad der Veisalgia als auch den Symptomen optimal angepasst werden.

Das Analgetikum ist vorzugsweise ausgewählt aus der Gruppe: Acetylsalicylsäure, Ibuprofen, Acetaminophen, Fenoprofen, Mefenaminsäure, Naproxen, Kodein oder Tolfenaminsäure.

Es hat sich als vorteilhaft erwiesen, gut untersuchte und standardisierte Analgetika zu verwenden. Analgetika mit Koffein sind bekannt und leicht zugänglich. Ihre Wechselwirkungen und Nebenwirkungen sind bestens dokumentiert und können daher im Rahmen der Erfindung im Rahmen fachüblicher Routinemassnahmen berücksichtigt werden. Im Rahmen der Erfindung anwendbare Handelsproduckte sind beispielsweise Contraschmerz® plus oder Alcacyl® Extra.

Das Antazidum kann aus der Gruppe: Aluminiumhydroxid, Magnesiumhydroxid, Kalziumcarbonat, Magnesiumcarbonat oder Aluminium-Magnesium-Silikathydrat, ausgewählt sein. Auch eine Kombination verschiedener Antazida ist möglich.

Durch die Verwendung eines Antazidums wird die Gefahr der Magenübersäuerung reduziert. Dies kann insbesondere bei der Einnahme von Ibuprofen von Bedeutung sein. Ibuprofen ist für seine magenreizende Wirkung bekannt. Gleichzeitig kann das Antazidum aber auch die Bioverfügbarkeit von Mineralien wie beispielsweise Kalzium oder Magnesium zusätzlich erhöhen.

Die erste und zweite Komponente des Therapiekits können gemeinsamen oder zeitlich versetzt mindestens 4 Stunden nach dem Alkoholkonsum verabreicht werden. Beispielsweise kann die erste Komponente 4 Stunden nach dem Alkoholkonsum und die zweite Komponente eine Stunde später verabreicht werden. Eine zeitlich versetzte Einnahme von bis zu 2 Stunden ist denkbar.

Die zeitgleiche Einnahme hat den Vorteil, dass der therapeutische Effekt verbessert wird. Die zeitlich versetzte Einnahme hat den Vorteil, dass eine Einnahme in Abhängigkeit der zu behandelnden Symptome erfolgen kann.

Eine weitere gemeinsame oder zeitlich versetzte Verabreichung der Komponenten kann mindestens 6 Stunden nach dem Alkoholkonsum erfolgen. Dies hat ebenfalls eine verbesserte therapeutische Wirkung zur Folge.

Die Verabreichung der ersten Komponente kann alternativ auch mindestens einmal vor dem Alkoholkonsum und während des Alkoholkonsums erfolgen. Es ist auch möglich, dass die Zusammensetzung zusätzlich nur vor dem Alkoholkonsum oder nur während des Alkoholkonsums verabreicht wird.

Die Einnahme der ersten Komponente vor dem Alkoholkonsum hat den Vorteil, dass die Nährstoffreserven im Körper aufgefüllt werden können. Die Einnahme während des Alkoholkonsums verringert den Verlust dieser Nährstoffe während dieser Zeit. Je nach getrunkener Alkoholmenge kann dies dem Auftreten von Veisalgia vorbeugen bzw. die Ausprägung der Symptome verringern.

Die Erfindung betrifft auch ein Analgetikum zur Behandlung von Veisalgia durch gemeinsame oder zeitlich versetzte Verabreichung mit einer Zusammensetzung wie oben beschrieben.

Das Analgetikum ist dabei vorzugsweise Acetylsalicylsäure, Ibuprofen, Acetaminophen, Fenoprofen, Mefenaminsäure, Naproxen, Kodein oder Tolfenaminsäure.

Durch eine gemeinsame Verabreichung mit der Zusammensetzung kann eine therapeutische Wirkung des Analgetikums verbessert werden. Die zeitlich versetzte Einnahme ermöglicht eine dem Krankheitsverlauf angepasste Behandlung.

Beispiel 1 zeigt eine bevorzugt Dosierung für eine erste Komponente und eine zweite Komponente für eine Einnahme nach dem Alkoholkonsum.

### Beispiel 1

| **Zusammensetzung (erste Komponente)** Brausetablette oder lösliches Pulver in 330 mL Wasser | [mg] | **Analgetikum (zweite Komponente)** Oral einnehmbare Tablette | **[mg]** |
|---|---|---|---|
| **Zuckerverbindung** | | Acetylsalicylsäure | 500 |
| Fruktose | 10'000 | Koffein | 50 |
| Dextrose* | 5'000 | | |
| **Mineralien** | | | |
| Kaliumchlorid | 660 | | |
| Natriumcitrat | 200 | | |
| Magnesiumcarbonat | 125 | | |
| Kalziumcarbonat | 266 | | |
| **Entgiftung** | | | |
| Betainhydrochlorid | 500 | | |
| Silybum marianum | 133 | | |
| Zingiberis rhizoma | 500 | | |
| **Zellschutz** | | | |
| Vitamin C | 80 | | |
| L-Cystein | 300 | | |
| N-Acetyl-L-cystein | 200 | | |
| Glutathion | 200 | | |
| Vitamin E | 12 | | |
| Phosphatidylserin | 100 | | |
| **Neurotransmitter** | | | |
| L-Tryptophan | 500 | | |
| L-Tyrosin | 166 | | |
| L-Glutamin | 3'000 | | |
| L-D-Phenylalanin (Racemat) | 333 | | |
| Dihydromyricetin | 300 | | |
| **Zellfunktion** | | | |
| Vitamin B₂(Riboflavin) | 1.4 | | |
| Vitamin B₁(Thiamin) | 1.1 | | |
| Vitamin B₃ (Nicotinamid)* | 18 | | |
| Vitamin B₁₂ (Cobolamin) | 0.0025 | | |
| Vitamin B₆(Pyridoxin) | 1.4 | | |
| Vitamin B₅(Pantothensäure) | 6 | | |

| **Spurenelemente** | | | |
|---|---|---|---|
| Selen | 0.018 | | |
| Zink | 5 | | |
| Folsäure | 0.2 | | |

| | | | |
|---|---|---|---|
| * Alternativ können 0 g Dextrose enthalten sein ** Alternativ sind auch 16 mg oder 17 mg möglich | | | |

Die erste Komponente in Beispiel 1 kann dabei allein, gemeinsam mit der zweiten Komponente oder zeitlich versetzt mit der zweiten Komponente eingenommen werden. Alternativ kann die erste Komponente allein auch vor und/oder während der Alkoholkonsumierung verabreicht werden.

Anhand der Figur, welche lediglich ein Dosierungsbeispiel darstellt, wird die Erfindung im Folgenden näher erläutert. Es zeigt:

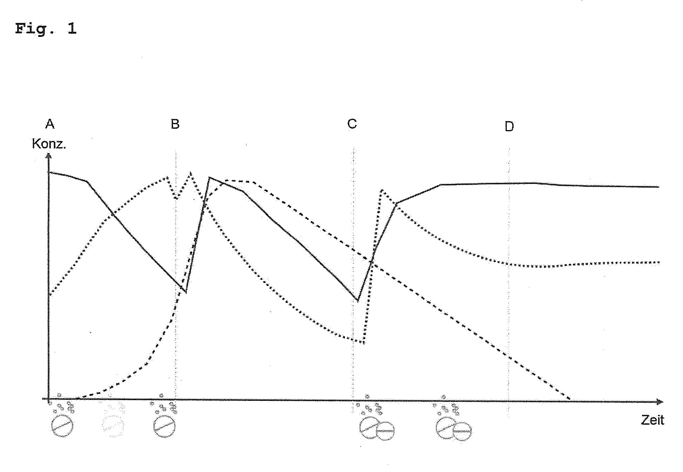

- Figur 1:: Eine erfindungsgemässe Dosierung der Zusammensetzung und eine schematische Darstellung des Konzentrationsverlaufs der im Körper vorhandenen Substanzen.

Figur 1 zeigt schematisch den Konzentrationsverlauf der im Körper vorhandenen Substanzen in Abhängigkeit von einer erfindungsgemässen Dosierung der Zusammensetzung. Die Kurve 3 kennzeichnet den Verlauf der Mineralstoffe während des Trinkens, gekennzeichnet durch das Zeitintervall A-B, während des Schlafens, gekennzeichnet durch das Zeitintervall B-C, während des Auftretens von Veisalgia-Symptomen, gekennzeichnet durch das Zeitintervall C-D und nach der Veisalgia D. Kurve 4 kennzeichnet dementsprechend der Verlauf des Blutzuckers und Kurve 5 zeigt die relative Konzentration an Giftstoffe im Blut an. Als Giftstoffe sind insbesondere Abbauprodukte aufgrund des Alkoholmetabolismus, wie beispielsweise Acetaldehyd und Acetat, zu verstehen. Eine Darreichungsform der Zusammensetzung 1a wird vor dem Alkoholkonsum A eingenommen. Die Konzentration der Mineralstoffe 3 im Blut erreicht einen maximalen Wert. Während des Alkoholkonsums A-B fällt die Konzentration an Mineralstoffen 3 ab. Der Blutzuckerwert 4 steigt, auch aufgrund der Zufuhr der alkoholischen Getränke. Die Konzentration an Giften 5 steigt weiter. Optional kann während des Alkoholkonsums A-B die Zusammensetzung 1b ein weiteres Mal eingenommen werden. Nach dem Alkoholkonsum B wird die Zusammensetzung 1c ein weiteres Mal eingenommen. Dadurch nimmt in der Anfangszeit der Schlafphase B-C die Konzentration an Mineralstoffen 3 wieder zu, fällt aber im späteren Verlauf durch den Verbrauch in biologischen Prozessen, insbesondere im Zusammenhang mit dem Abbau von Giftstoffen 5, wieder ab. Auch die Konzentration an Blutzucker 4 fällt nach kurzem Anstieg wieder ab. Aufgrund des Alkoholmetabolismus steigt die Konzentration an Giften 5 im Blut während der Schlafphase B-C auf einen maximalen Wert an. Danach wird die Konzentration an Gifte 5 stetig abgebaut. Der Zusammenhang zwischen den Abbau an Giftstoffen 5 und den Verlust an Nährstoffen 3 ist an der nahezu parallel abfallenden Kurve verdeutlicht. Direkt nach dem Aufwachen C wird die Zusammensetzung 1d zusammen mit einem Analgetikum 2a eingenommen, um die Behandlung von Veisalgia zu beschleunigen sowie der Symptomentwicklung entgegenzuwirken und eine möglichst rasche Linderung zu erzielen. Durch die Einnahme der Zusammensetzung 1d steigt der Blutzucker 4 wieder an, ebenso erhöht sich die Konzentration an Mineralstoffen 3. 2 Stunden nach dem Aufwachen C wird die Zusammensetzung 1e ein weiteres Mal mit einem Analgetikum 2b eingenommen. Dadurch wird auch der Abbau der Gifte 5 unterstützt. Nach Ausheilung der Veisalgia D, erreichen die Konzentration an Mineralstoffen 3 und an Blutzucker 4 einen annähernd konstanten Wert. Die Gifte 5 sind nahezu vollständig abgebaut.

## Patentansprüche

1. Zusammensetzung zur Anwendung bei der Therapie von Veisalgia, umfassend:
- mindestens einen Zucker oder eine Zuckerverbindung, insbesondere ausgewählt aus der Gruppe: Fruktose; Saccharose; Glucose;
- Mineralsalze; insbesondere ein Kaliumsalz, ein Natriumsalz, ein Magnesiumsalz und ein Kalziumsalz; besonders bevorzugt Kaliumchlorid, Natriumcitrat, Magnesiumcarbonat und/oder Kalziumcarbonat;
- mindestens eine Verbindung zum Zellschutz, insbesondere ein Antioxidans ausgewählt aus der Gruppe: Ascorbinsäure; Tocopherol; Glutathion-Präkursor, insbesondere N-Acyl-L-Cystein, L-Cystein und/oder Glutaminsäure; Glutathion und/oder Phospahtidylserin;
- mindestens eine Verbindung zur Unterstützung der Zellfunktion, insbesondere ausgewählt aus der Gruppe: Nicotinamid; Nicotinsäure; Riboflavin; Pantothensäure; Pyridoxin; Thiamin; Vitamin B₁₂;
- mindestens eine Verbindung zur Förderung der Entgiftung, insbesondere ausgewählt aus der Gruppe: Betaine; Silybum marianum; Zingiberis rhizoma;
- mindestens einen Neurotransmitter, insbesondere ausgewählt aus der Gruppe: L-Tyrosin; L-Tryptophan; L-Phenylalanin; L-D-Phenylalanin (Racemat); L-Glutamin; Dihydromyricetine;
- mindestens ein Spurenelement, insbesondere Selen und/oder Zink;
- Folsäure;
- optional: weitere Spurenelemente, insbesondere Biotin, Mangan und/oder Molybdän;
- optional: ein stimulierendes Alkaloid, insbesondere Koffein;
wobei die Zusammensetzung kein Analgetikum enthält.

2. Zusammensetzung nach Anspruch 1, wobei die Zusammensetzung mindestens ein weiteres Additiv ausgewählt aus der Gruppe: Aroma; Farbstoffe; Zerfallsbeschleuniger, insbesondere Natriumhydrogencarbonat; Säuerungsmittel, insbesondere Zitronensäure, enthält.

3. Zusammensetzung nach Anspruch 1 oder 2, wobei die Zusammensetzung als Tablette, insbesondere Brausetablette; Kapsel; Suspension; lösbares Pulver; Fertiggetränk oder Depotformulierung vorliegt.

4. Zusammensetzung zur Anwendung bei der Therapie von Veisalgia, wobei die Zusammensetzung
- mindestens einen Zucker oder eine Zuckerverbindung, insbesondere ausgewählt aus der Gruppe: Fruktose; Saccharose; Glucose;
- Mineralsalze; insbesondere ein Kaliumsalz, ein Natriumsalz, ein Magnesiumsalz und ein Kalziumsalz; besonders bevorzugt Kaliumchlorid, Natriumcitrat, Magnesiumcarbonat und/oder Kalziumcarbonat;
- mindestens eine Verbindung zum Zellschutz, insbesondere ein Antioxidans ausgewählt aus der Gruppe: Ascorbinsäure; Tocopherol; Glutathion-Präkursor, insbesondere N-Acyl-L-Cystein, L-Cystein und/oder Glutaminsäure; Glutathion und/oder Phospahtidylserin.
- mindestens eine Verbindung zur Unterstützung der Zellfunktion, insbesondere ausgewählt aus der Gruppe: Nicotinamid; Nicotinsäure; Riboflavin; Pantothensäure; Pyridoxin; Thiamin; Vitamin B₁₂;
- mindestens eine Verbindung zur Förderung der Entgiftung, insbesondere ausgewählt aus der Gruppe: Betaine; Silybum marianum; Zingiberis rhizoma;
- mindestens einen Neurotransmitter, insbesondere ausgewählt aus der Gruppe: L-Tyrosin; L-Tryptophan; L-Phenylalanin; L-D-Phenylalanin (Racemat); L-Glutamin, Dihydromyricetine;
- mindestens ein Spurenelement, insbesondere Selen und/oder Zink;
- Folsäure;
- optional: weitere Spurenelemente, insbesondere Biotin, Mangan und/oder Molybdän;
- optional: ein stimulierendes Alkaloid, insbesondere Koffein
umfasst zur Verabreichung mindestens zweimal nach dem Alkoholkonsum.

5. Zusammensetzung nach Anspruch 4, wobei die Verabreichung mindestens einmal direkt nach dem Alkoholkonsum und zwei weitere Male innerhalb von 12 Stunden erfolgt, wobei zwischen den Einnahmen jeweils eine Pause von mindestens 2 Stunden liegt.

6. Zusammensetzung nach einem der Ansprüche 4 oder 5 zur mindestens einmal zusätzlichen Verabreichung der Zusammensetzung vor dem Alkoholkonsum und/oder während des Alkoholkonsums.

7. Zusammensetzung nach einem der Ansprüche 4 bis 6, zur Verabreichung der Zusammensetzung mindestens 4 Stunden nach dem Alkoholkonsum zusammen mit einem Analgetikum.

8. Zusammensetzung nach einem der vorherigen Ansprüche, wobei die Zusammensetzung
- 5 bis 30 g Zucker oder Zuckerverbindungen;
- 100 bis 1500 mg der Mineralsalze;
- 10 bis 900 mg Verbindungen zum Zellschutz;
- 3 bis 7 g der Neurotransmitter;
- 1 bis 30 mg Verbindungen zur Unterstützung der Zellfunktion;
- 200 bis 1200 mg Verbindungen zur Förderung von Entgiftung;
- 1 bis 7 mg der Spurenelemente und Folsäure; und
- optional: 2 bis 10 mg Biotin; 1 bis 6 mg Mangan; 50-300 mg Molybdän; und/oder 20-100 mg Koffein
enthält.

9. Therapiekit zur Anwendung bei der Behandlung von Veisalgia, umfassend mindestens zwei separat verabreichbare Komponenten, wobei die erste Komponente eine Zusammensetzung mit
- mindestens einem Zucker oder einer Zuckerverbindung, insbesondere ausgewählt aus der Gruppe: Fruktose; Saccharose; Glucose;
- Mineralsalzen; insbesondere einem Kaliumsalz, einem Natriumsalz, einem Magnesiumsalz und einem Kalziumsalz; besonders bevorzugt Kaliumchlorid, Natriumcitrat, Magnesiumcarbonat und/oder Kalziumcarbonat;
- mindestens einer Verbindung zum Zellschutz, insbesondere einem Antioxidans ausgewählt aus der Gruppe: Ascorbinsäure; Tocopherol; Glutathion-Präkursor, insbesondere N-Acyl-L-Cystein, L-Cystein und/oder Glutaminsäure; Glutathion und/oder Phospahtidylserin;
- mindestens einer Verbindung zur Unterstützung der Zellfunktion, insbesondere ausgewählt aus der Gruppe: Nicotinamid; Nicotinsäure; Riboflavin; Pantothensäure; Pyridoxin; Thiamin; Vitamin B₁₂;
- mindestens einer Verbindung zur Förderung der Entgiftung, insbesondere ausgewählt aus der Gruppe: Betaine; Silybum marianum; Zingiberis rhizoma;
- mindestens einem Neurotransmitter, insbesondere ausgewählt aus der Gruppe: L-Tyrosin; L-Tryptophan; L-Phenylalanin; L-D-Phenylalanin (Racemat); L-Glutamin; Dihydromyricetine;
- mindestens einem Spurenelement, insbesondere Selen und/oder Zink;
- Folsäure;
- optional: weiteren Spurenelementen, insbesondere Biotin, Mangan und/oder Molybdän;
- optional: einem stimulierenden Alkaloid, insbesondere Koffein;
umfasst und kein Analgetikum enthält;
und wobei die zweite Komponente ein Analgetikum und optional ein Antazidum und/oder optional ein stimulierendes Alkaloid, vorzugsweise Koffein, enthält.

10. Therapiekit nach Anspruch 9, wobei das Analgetikum ausgewählt ist aus der Gruppe: Acetylsalicylsäure; Ibuprofen; Acetaminophen; Fenoprofen; Mefenaminsäure; Naproxen; Kodein; Tolfenaminsäure.

11. Therapiekit nach einem der Ansprüche 9 oder 10, wobei das Antazidum ausgewählt ist aus der Gruppe: Aluminiumhydroxid; Magnesiumhydroxid; Kalziumcarbonat; Magnesiumcarbonat; Aluminium-Magnesium-Silikathydrat.

12. Therapiekit nach einem der Ansprüche 9 bis 11 zur gemeinsamen oder zeitlich versetzten Verabreichung der Komponenten mindestens 4 Stunden nach dem Alkoholkonsum.

13. Therapiekit nach einem der Ansprüche 9 bis 12 zur gemeinsamen oder zeitlich versetzten Verabreichung der Komponenten mindestens 6 Stunden nach dem Alkoholkonsum, insbesondere zusätzlich zur gemeinsamen oder zeitlich versetzten Verabreichung der Komponenten mindestens 4 Stunden nach dem Alkoholkonsum.

14. Therapiekit nach Anspruch 9 bis 13 zur Verabreichung der ersten Komponente mindestens einmal vor dem Alkoholkonsum und/oder während des Alkoholkonsums, insbesondere zusätzlich zur gemeinsamen oder zeitlich versetzten Verabreichung der Komponenten mindestens 4 Stunden und/oder 6 Stunden nach dem Alkoholkonsum.

15. Analgetikum zur Behandlung von Veisalgia durch gemeinsame oder zeitlich versetzte Verabreichung mit einer Zusammensetzung gemäss einem der Ansprüche 1 bis 3.
